# EUROPÄISCHE PATENTANMELDUNG

(11) **EP 0 583 752 A1**
(43) Veröffentlichungstag der Anmeldung: **23.02.1994**
(21) Anmeldenummer: 93112988.6
(22) Anmeldetag: 13.08.1993
(51) Int. Cl.: C07H 9/04

(54) **Verfahren zur Herstellung von 1,2:5,6-Di-O-isopropyliden derivaten von Monosacchariden, insbesondere von 1,2-5,6-Diaceton-D-glucose**

(30) Priorität: 14.08.1992 DE 4227022
(71) Anmelder: BOEHRINGER INGELHEIM KG, D-55216 Ingelheim (DE); BOEHRINGER INGELHEIM INTERNATIONAL GmbH, D-55216 Ingelheim am Rhein (DE)
(72) Erfinder: Klingler, Franz, Dr. Ing., D-6103 Griesheim (DE); Christmann, Albrecht, Dr.Dipl.-Chem., D-6507 Ingelheim am Rhein (DE)

(57) **Zusammenfassung**

Die vorliegende Erfindung betrifft ein Verfahren zur Herstellung von 1,2:5,6-Di-O-isoporpylidenderivaten von Monosacchariden, die zwei sterisch benachbarte cis-ständige Hydroxylgruppen aufweisen, insbesondere von 1,2-5,6-Diaceton-D-glucose aus D-Glucose und Aceton.

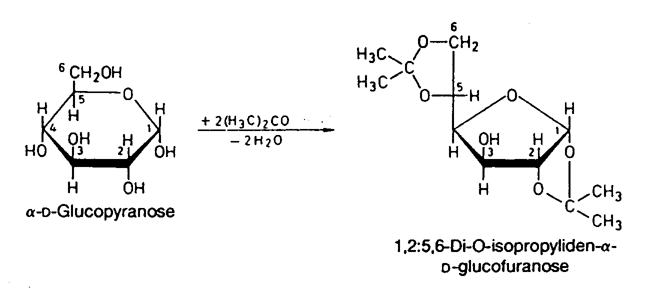

## Beschreibung

Die vorliegende Erfindung betrifft ein verbessertes Verfahren zur Herstellung von 1,2:5,6-Di-O-isopropylidenderivaten von Monosacchariden, die zwei sterisch benachbarte cis-ständige Hydroxylgruppen aufweisen, insbesondere von 1,2-5,6-Diaceton-D-glucose (1,2:5,6-Di-O-isopropyliden-α-D-glucofuranose) 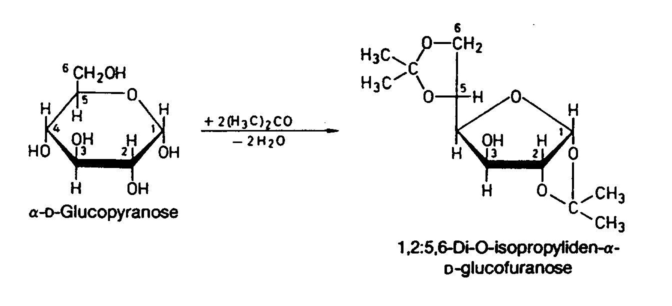
aus D-Glucose und Aceton.

1,2-5,6-Diaceton-D-glucose stellt ein zentrales Zwischenprodukt für zahlreiche andere Glucoseabkömmlinge dar, die u.a. als Arzneimittel große Bedeutung haben. Als Beispiele seien lediglich das 2-Deoxy-D-riboseanilid oder Amiprilose genannt.

Daneben kann 1,2-5,6-Diaceton-D-glucose als chrialer Ligand in Komplexen eingesetzt werden, die enantioselektive Reaktionen ermöglichen [F.D. Klingler und M. Psiorz, Chimicaoggi 1992, 47].

Diese zentrale Rolle der 1,2-5,6-Diaceton-D-glucose ist dafür verantwortlich, daß ein jährlicher Bedarf in Tonnengrößenordnungen an diesem Zwischenprodukt besteht.

Aus dem Stand der Technik ist allgemein bekannt, daß Monosacharide, die zwei sterisch benachbarte, cis-ständige OH-Gruppen enthalten, mit Aldehyden oder Ketonen in Gegenwart von Schwefelsäure, Zinkchlorid oder Phosphor-(V)-oxid zu den entsprechenden Acetalen umgesetzt werden können (E. Fischer, 1895).

So ist die 1,2:5,6-Di-O-isopropyliden-α-D--glucofuranose (Diaceton-α-D-glucose) durch Umsetzung von D-Glucose mit Aceton in Gegenwart von Schwefelsäure erhältlich. Dabei muß - zur Erzielung hoher Umsätze - das aus der Ketalisierung resultierende Wasser gebunden bzw. aus dem Reaktionsgemisch entfernt werden.

Als weitere Katalysatoren sind aus dem Stand der Technik u.a. auch Jod, Gips oder Molekularsiebe bekannt. Jedoch ist die Verwendung der bislang als geeignet bekannten Katalysatoren - nicht nur hinsichtlich der Reaktionen im industriellen Maßstab - mit gravierenden Nachteilen verbunden, von denen beispielhaft die folgenden genannt sein sollen:
- bei der Verwendung von Mineralsäuren oder Phosphorpentoxid müssen große Mengen dieser Agenzien eingesetzt werden, was zum einen nur einen geringen Durchsatz erlaubt und zum anderen große Entsorgungsprobleme der aus der anschließend notwendigen Neutralisation resultierenden Salze mit sich bringt;
- bei der Verwendung von Jod sind große Mengen Lösungsmittel erforderlich, die ebenfalls nur einen geringen Durchsatz erlauben;
- beim Einsatz eines zusätzlichen Lösungsmittels, welches in der Lage ist, ein Azeotrop mit Wasser zu bilden, wird eine weitere Vergrößerung des Volumens des Reaktorgefäßes erforderlich - zudem geht der Einsatz eines Schleppmittels - wie beispielsweise Pentan - mit einer Siedepunktserniedrigung einher, womit die Reaktionstemperatur limitiert wird und womit sich die Reaktionszeit entsprechend verlängert;
- der Einsatz fester Katalysatoren bereitet durch die ebenfalls stattfindenden Karamelisierungsreaktionen ebenfalls Schwierigkeiten - außerdem ist beispielsweise die Wiederaufbereitung von Ionenaustauschern mit einem sehr großen Aufwand verbunden;
- daneben haftet vielen Umsetzungen der Nachteil an, daß durch die - in ihrem Ausmaß von den jeweiligen Reaktionsbedingungen abhängigen - Nebenreaktionen - wie z.B. die Selbstkondensation des Acetons - z.T. teerartige Nebenprodukte entstehen, die einerseits die Wirksamkeit des Katalysators beeinträchtigen und andererseits zu einer unerwünschten Verunreinigung des Reaktionsproduktes führen, die teilweise lediglich im Rahmen einer chromatographischen Reinigung wieder entfernbar sind.

Das erfindungsgemäße Verfahren ermöglicht somit die Herstellung von Diacetonglucose in Gegenwart sauer reagierenden Katalysatoren, wie z.B. Lewis-Säuren - bevorzugt Klompexverbindungen des Bortrifluorids, Aluminiumhalogenide - wie z.B. Aluminium(III)-chlorid -, Kupfersalze - wie z.B. Kupfer(II)-chlorid oder -bromid -, Eisensalze - wie z.B. Eisen(III)-chlorid oder -bromid -, Zinnsalze oder Halogenide seltener Erden, wobei der Bortrifluorid-Diethylether-Komplex (Bortrifluorid-Etherat) (eingesetzt in 1 % bezogen auf die eingesetzte Glucose) besonders bevorzugt wird - ohne Zusatz von wasserentziehenden Agenzien oder anderen Trocknungszusatz und ohne den Zusatz von weiteren Lösungsmitteln als Schleppmittel.

Dazu wird die α-D-Glucose in einem geeigneten Autoklaven vorgelegt und mit der Mischung aus Aceton und Bortrifluorid-Etherat versetzt. Die Reaktionsmischung wird anschließend erhitzt bis sich im Reaktionsgefäß ein Druck von mindestens 2,5 bar - vorzugsweise ein Druck im Bereich von 2,5 bis 10 bar und besonders bevorzugt ein Druck im Bereich von 2,5 bis 5,5 bar - aufgebaut hat. Anschließend werden bei einer Temperatur im Bereich von 80 bis 130°C bevorzugt 85 bis 120°C und besonders bevorzugt 88 bis 115°C die unter diesen Reaktionsbedingungen flüchtigen Bestandteile der Mischung abdestilliert, wobei in dem Maße, in dem das abdestillierte Reaktionsmedium als Destillat anfällt Aceton zur Reaktionsmischung zugefügt wird.

Die Destillation wird so lange durchgeführt, bis die Menge an Destillat mindestens das 1 2/3-fache des ursprünglich eingesetzten Volumens an Aceton erreicht hat. Das verbliebene Reaktionsgemisch wird danach im Vakuum bei einer Temperatur im Bereich von 30 bis 70°C bevorzugt 35 bis 50°C und besonders bevorzugt bei einer Temperatur von 40°C auf ca. die Hälfte seines ursprünglichen Volumens eingeengt.

Im anschließenden Reaktionsschritt wird das Reaktionsgemisch mit der wässerigen Lösung einer Base vorzugsweise mit einer wässerigen Lösung eines Alkalihydroxyds und besonders bevorzugt mit 2 N Natronlauge und Aceton verdünnt und gerührt.

Danach wird das Reaktionsgemisch erneut im Vakuum bei einer Temperatur im Bereich von 30 bis 70°C, bevorzugt 35 bis 50°C und besonders bevorzugt bei einer Temperatur von 40°C eingeengt, bis alle unter diesen Bedingungen flüchtigen organischen Bestandteile weitgehendst abdestilliert worden sind. Nach dem Abkühlen wird der verbliebene Rückstand mit einem organischen Extraktionsmittel - vorzugsweise einem aliphatischen oder aromatischen Kohlenwasserstoff - wie z.B. Pentan, Hexan, Erdölfraktionen, Toluol oder Xylol - oder einem Ether - wie z.B. Diethylether, Methyl-tert.-butylether - oder einem halogenierten Kohlenwasserstoff, worunter Dichlormethan besonders bevorzugt wird - extrahiert.

Die vereinigten Extrakte werden im Vakuum eingeengt, den verbliebenen Rückstand mit einem organischen Lösungsmittel vorzugsweise einem aliphatischen Kohlenwasserstoff besonders bevorzugt Cyclohexan versetzt und - unter Nomaldruck - auf eine Temperatur im Bereich von 65 bis 80°C - bevorzugt auf 70°C erhitzt - und anschließend auf eine Temperatur von ca. 10°C abkühlt, wobei die 1,2-5,6-Diaceton-α-D-glucose als kristalliner Feststoff ausfällt.

Anschließend wird das Kristallisat isoliert und getrocknet.

Die eingangs genannten Aufgaben werden durch das im Beispiel beschriebene Verfahren gelöst.
Verschiedenartige, andere Ausgestaltungen des Verfahrens werden für den Fachmann aus der vorliegenden Beschreibung ersichtlich. Es wird jedoch ausdrücklich darauf hingewiesen, daß die Beispiele und die diesen zugeordnete Beschreibung lediglich zum Zweck der Erläuterung und Beschreibung vorgesehen und nicht als Einschränkung der Erfindung anzusehen sind. Insbesondere wird darauf hingewiesen, daß die in den Beispielen zur Herstellung von 1,2:5,6-Di-O--isopropyliden-α-D-glucofuranose beschriebene Synthesefolge auf die Herstellung anderer Isopropyliden-Zucker übertragen werden kann.

Als in Frage kommende Ausgangsstoffe seien beispielsweise folgende Monosaccharide genannt:
D-Galactose, L- sowie D-Arabinose, D-Xylose, D-Mannose, D-Ribose, D-Mannit und L-Ascorbinsäure.

### Beispiel

3,66 kg (20,32 Mol) wasserfreie α-D-Glucose werden in ein mit Schutzgas gespültes Reaktionsgefäß eingewogen und mit einer Mischung aus 75 l Aceton und 31 ml Bortrifluorid-Diethylether-Komplex versetzt. Unter einem Druck, der im Bereich von 1,6 bis 4,4 bar liegt, werden die unter diesen Bedingungen in einem Temperaturintervall von 88 bis 115°C flüchtigen Bestandteile des Reaktionsgemisches abdestilliert, wobei in dem Maße, in dem das Destillat anfällt, dem Destillationsrückstand Aceton zugeführt wird.

Nach einer Destillationszeit von 4 h wird die Zuführung von Aceton unterbrochen und unter vermindertem Druck werden bei einer Temperatur von 40°C 35 l Aceton abdestilliert. Danach läßt man den Destillationsrückstand auf Raumtemperatur abkühlen und fügt 3,1 1 2N Natronlauge sowie 19 l destilliertes Wasser zu.

Anschließend wird 10 Min. nachgerührt und das eingesetzte Aceton danach unter vermindertem Druck (Wasserringpumpenvakuum) aus dem Reaktionsgemisch weitgehendst abdestilliert. Anschließend läßt man den Destillationsrückstand auf Raumtemperatur abkühlen, fügt 19 l Dichlormethan und rührt 15 Min. kräftig nach. Man läßt das Extraktionsgemisch über einen Zeitraum von 30 Min. absitzen und trennt danach die organische Phase ab.

Die Extraktion wird noch zweimal unter Verwendung von jeweils 13 l Dichlormethan wiederholt und die vereinigten Extrakte werden anschließend im Vakuum vom Extraktionsmittel befreit. Der Rückstand wird mit 22,5 l Cyclohexan versetzt und auf eine Temperatur von 70°C erhitzt. Danach wird die Lösung auf 10°C abgekühlt und bei dieser Temperatur 2 h lang gerührt. Die daraus resultierende Kristallsuspension wird filtriert und der kristalline Rückstand mit Cyclohexan nachgewaschen und bei einer Temperatur von 40°C unter vermindertem Druck getrocknet. Man erhält auf diese Weise 3,28 kg (62 % d. Th.) der 1,2:5,6-Di-O-isopropyliden-α-D-glucofuranose in Form eines kristallinen Festkörpers.

## Patentansprüche

1. Verfahren zur Herstellung von 1,2:5,6-Di-O-isopropylidenderivaten von Monosacchariden, die zwei sterisch benachbarte cis-ständige Hydroxylgruppen aufweisen, dadurch gekennzeichnet, daß man das Monosaccharid, in Gegenwart einer Lewis-Säure mit Aceton bei erhöhter Temperatur und unter erhöhtem Druck umsetzt, die unter den gewählten Reaktionsbedingungen flüchtigen Bestandteile abdestilliert und das abdestillierte Volumen durch Aceton ersetzt, das Reaktionsgemisch unter vermindertem Druck einengt, mit der wässerigen Lösung einer Base versetzt, das resultierende Reaktionsgemisch mit einem organischen Extraktionsmittel extrahiert, die vereinigten Extrakte einengt, den verbliebenen Rückstand in einem organischen Lösungsmittel aufnimmt, die resultierende Lösung oder Suspension erwärmt und das beim nachfolgenden Abkühlen ausfallende Reaktionsprodukt isoliert.

2. Verfahren zur Herstellung von 1,2-5,6-Diaceton-D--glucose nach Anspruch 1, dadurch gekennzeichnet, daß man α-D-Glucose in Gegenwart einer Lewis-Säure mit Aceton bei einer Temperatur im Bereich von 80 bis 120°C bei einem Druck von mindestens 2,5 bar umsetzt, dabei die unter diesen Reaktionsbedingungen flüchtigen Bestandteile abdestilliert und die Menge des Destillats durch Aceton ersetzt bis ca. das 1 2/3-fache des ursprünglichen Reaktionsvolumens durch Aceton substituiert worden ist, anschließend das Reaktionsgemisch unter vermindertem Druck bei einer Temperatur im Bereich von 30 bis 70°C einengt, mit der wässerigen Lösung einer Base versetzt, mit einem organischen Extraktionsmittel extrahiert, die Extrakte einengt, den verbliebenen Rückstand in einem organischen Lösungsmittel aufnimmt, auf eine Temperatur im Bereich von 65 bis 80°C erhitzt und die beim anschließenden Abkühlen ausfallenden Kristalle isoliert.

3. Verfahren nach Anspruch 2, dadurch gekennzeichnet, daß man α-D-Glucose in Gegenwart einer Komplexverbindung des Bortrifluorids, von Aluminium(III)-halogeniden, Kupfer-, Eisen- oder Zinnsalzen oder von Halogeniden der seltenen Erden mit Aceton bei einer Temperatur im Bereich von 85 bis 120°C bei einem Druck im Bereich von 2,5 bis 10 bar umsetzt, dabei die unter diesen Bedingungen flüchtigen Bestandteile abdestilliert und die Menge des Destillats durch Aceton ersetzt bis ca. das 1 2/3-fache des ursprünglichen Reaktionsvolumens durch Aceton substituiert worden ist, anschließend das Reaktionsgemisch unter vermindertem Druck bei einer Temperatur im Bereich von 35 bis 50°C einengt, mit der wässerigen Lösung eines Alkalihydroxyds versetzt, die Reaktionsmischung unter vermindertem Druck bei einer Temperatur im Bereich von 35 bis 50°C einengt, mit einem aliphatischen oder aromatischen Kohlenwasserstoff oder einem Ether oder einem halogenierten Kohlenwasserstoff extrahiert, die Extrakte einengt, den verbliebenen Rückstand mit einem Kohlenwasserstoff versetzt, auf eine Temperatur im Bereich von 65 bis 80°C erhitzt und die beim abschließenden Abkühlen ausfallenden Kristalle isoliert.

4. Verfahren nach Anspruch 3, dadurch gekennzeichnet, daß man α-D-Glucose in Gegenwart von Bortrifluorid-Etherat mit Aceton bei einer Temperatur im Bereich von 88 bis 115°C bei einem Druck im Bereich von 2,5 bis 5,5 bar umsetzt, dabei die unter diesen Bedingungen flüchtigen Bestandteile abdestilliert und die Menge des Destillats durch Aceton ersetzt bis ca. das 1 2/3-fache des ursprünglichen Reaktionsvolumens durch Aceton substituiert worden ist, anschließend das Reaktionsgemisch unter vermindertem Druck bei einer Temperatur im Bereich von 40°C einengt, mit 2N Natronlauge versetzt, die Reaktionsmischung unter vermindertem Druck bei einer Temperatur im Bereich von 40°C einengt, mit einem Dichlormethan extrahiert, die Extrakte einengt, den verbliebenen Rückstand mit Cyclohexan versetzt, auf eine Temperatur im Bereich von 70°C erhitzt und die beim anschließenden Abkühlen auf eine Temperatur von 10°C ausfallenden Kristalle isoliert.
